# EUROPEAN PATENT SPECIFICATION

(11) **EP 0 664 798 B1**
(45) Date of publication and mention of the grant of the patent: **29.12.1999**
(21) Application number: 94921063.7
(22) Date of filing: 21.07.1994
(51) Int. Cl.: C07D 271/07, C07D 271/113, C07D 285/08, C07D 413/02, C07D 417/02, C07F 7/18, A01N 43/824, A01N 43/836, A01N 55/10

(54) **THIADIAZOLE DERIVATIVES AND THEIR USE AS FUNGICIDES OR INSECTICIDES**
THIADIAZOLDERIVATE UND IHRE VERWENDUNG ALS FUNGIZIDE ODER INSEKTIZIDE
DERIVES DE THIADIAZOLE UTILISES COMME FONGICIDES ET INSECTICIDES

(30) Priority: 13.08.1993 GB 9316929; 11.03.1994 GB 9404777
(43) Date of publication of application: 02.08.1995
(73) Proprietor: ZENECA LIMITED, London W1Y 6LN (GB)
(72) Inventor: MATTHEWS, Ian, Richard, Berkshire RG11 1HH (GB); BACON, David, Philip, Wokingham Berkshire RG11 1NZ (GB)
(74) Representative: Houghton, Malcolm John
(86) International application number: GB9401574
(87) International publication number: WO9505368

(56) References cited:
- EP-A- 0 254 426
- EP-A- 0 256 667
- EP-A- 0 623 604
- WO-A-94/10159

## Description

The present invention relates to novel thiadiazole derivatives, to processes for preparing them, to fungicidal compositions containing them and to methods of using them to combat fungi, especially fungal infections of plants.

Certain fungicidal and insecticidal derivatives of 5-membered heterocycles, including 1,3,4-thiadiazole, are described in EP-A-0256667. In these derivatives, the heterocycle is linked to a methyl 2-phenyl-3-methoxyacrylate group.

Certain fungicidal and insecticidal derivatives of 5-membered heterocycles, including 1,3,4-thiadiazoles, are also described in WO-A-9410159 and EP-A-0623604 ; these documents are only relevant under the terms of Article 54(3) EPC.

According to the present invention there is provided a compound having the general formula (I), or a stereoisomer thereof, wherein A is CH or N; B is OCH₃ or NHCH₃; and X is C₁₋₈ alkyl (optionally substituted with C₁₋₈ alkoxy, halo(C₁₋₈)alkoxy, C₁₋₈ alkoxy(C₁₋₈)alkoxy, C₁₋₈ alkylthio, C₃₋₆ alkenyloxy, C₃₋₆ alkynyloxy or tri(C₁₋₈) alkylsilyloxy), halo(C₁₋₈)alkyl, C₂₋₈ alkenyl, C₂₋₈ alkynyl or C₃₋₇ cycloalkyl(C₂₋₆)alkyl. Because the double bond of the group, BOC.C=A.OCH₃, is unsymmetrically substituted, the compounds of the invention may be obtained in the form of mixtures of (E)- and (Z)-geometric isomers. However, these mixtures can be separated into individual isomers, and this invention embraces such isomers and mixtures thereof in all proportions including those which consist substantially of the (Z)-isomer and those which consist substantially of the (E)-isomer. The (E)-isomer, in which the groups -COB and -OCH₃ are on opposite sides of the olefinic bond, is generally the more fungicidally active and forms a preferred embodiment of the invention. Of particular interest is the group, CH₃O₂C.C=CH.OCH₃, and especially its (E)-isomer.

The 5-membered ring in formula (I) is a 1,2,4-thiadiazole ring.

Unless otherwise stated, the term alkyl is used to mean an alkyl group containing from 1 to 8 and suitably from 1 to 6, for example 1 to 4, carbon atoms in the form of straight or branched chains. Examples are methyl, ethyl, n- and iso-propyl, n-, sec-, iso- and tert-butyl, n-pentyl, iso-amyl, 2-methylbutyl, n-hexyl and n-octyl.

Haloalkyl, and the haloalkyl part of haloalkoxy, include trihaloalkyl or partially or fully halogenated alkyl. Examples are trifluoro- and trichloromethyl, and pentafluoroethyl. By alkenyl and alkynyl are meant alkenyl and alkynyl groups containing from 2 to 8 and suitably from 3 to 6, for example 3 or 4, carbon atoms in the form of straight or branched atoms. Examples are allyl, 1 -propenyl and propargyl.

The alkyl part or parts of alkoxy, alkoxyalkoxy and alkylthio groups are straight or branched chains containing from I to 8, suitably from 1 to 6, for example 1 to 4, carbon atoms. Examples of these groups are methoxy, ethoxy, ethoxymethoxy, methoxymethoxy, methylthio and ethylthio.

The alkyl part of the cycloalkylalkyl group is a straight or branched chain and contains from 2 to 6, for example from 2 to 4, carbon atoms. The cycloalkyl part of this group contains from 3 to 7 carbon atoms. This group is, for example, 2-(cyclopropyl)eth-1-yl, 1-(cyclopropyl)eth-1-yl, 2-(cyclopropyl)prop-2-yl and 1-(cyclohexyl)eth-1-yl. The hydrocarbon part of alkenyloxy or alkynyloxy is in the form of a straight or branched chain and preferably contains 3 or 4 carbon atoms. Examples are allyloxy and propargyloxy. In one aspect the invention provides a compound of the general formula (I) wherein A is CH or N; B is OCH₃ or NHCH₃; and X is C₁₋₆ alkyl (optionally substituted with C₁₋₄ alkoxy, C₁₋₂ alkoxy(C₁₋₄)alkoxy or C₁₋₄ alkylthio), halo(C₁₋₆)alkyl, C₃₋₆ alkenyl, C₃₋₄ alkynyl or cyclopropyl(C₂₋₃)alkyl. Suitably A is N and B is OCH₃ or NHCH₃, but preferably A is CH and B is OCH₃.

In another aspect the invention provides a compound of the general formula (1.2) wherein X is C₁₋₅ alkyl or propenyl.

The present invention is illustrated by compounds of the general formula (I) listed in Tables 1 to 4 which follow. Throughout these Tables the group BOC.C=A.OCH₃ has the (E)-configuration.

**TABLE 1**

| | | | |
|---|---|---|---|
| Table 1 comprises 68 compounds of the general formula (I), wherein A is CH, B is OCH₃, and X has the value listed. | | | |

| Compound No | X | Compound No | X |
|---|---|---|---|
| 1 | CH₃ | 11 | CH₃CH₂(CH₃)CHCH₂ |
| 2 | CH₃CH₂ | 12 | CH₃ (CH₂)₂ (CH₃)CH |
| 3 | CH₃ (CH₂)₂ | 13 | CH₃CH₂(CH₃)₂C |
| 4 | (CH₃)₂CH | 14 | (CH₃)₂CH(CH₃)CH |
| 5 | CH₃(CH₂)₃ | 15 | (CH₃)₃CCH₂ |
| 6 | CH₃CH₂(CM₃)CH | 16 | CH₃ (CH₂)₅ |
| 7 | (CH₃)₂CHCH₂ | 17 | CH₃(CH₂)₇ |
| 8 | (CH₃)₃C | 18 | CH₃CH=CH |
| 9 | CH₃(CH₂)₄ | 19 | CH₂=CHCH₂ |
| 10 | (CH₃)₂CH(CH₂)₂ | 20 | CH₂=CH(CH₃)₂C |
| 21 | CH₂=CHCH₂(CH₃)₂C | 52 | C₂H₅S(CH₂)₂ |
| 22 | CH₃CH=CH(CH₃)₂C | 53 | (CH₃)₂CHO(CH₂)₂ |
| 23 | CCl₃ | 54 | (CH₃)₂CHS(CH₂)₂ |
| 24 | CF₃ | 55 | CH₃OC(CH₃)₂ |
| 25 | (CH₃CH₂)₂CH | 56 | CH₃SC(CH₃)₂ |
| 26 | C₂F₅ | 57 | CH₃OCH(CH₃) |
| 27 | CF₃(CF₂)₂ | 58 | CH₃SCH(CH₃) |
| 28 | (CF₃)₂CF | 59 | C₂H₅OCH(CH₃) |
| 29 | CF₃(CF₂)₃ | 60 | C₂H₅SCH(CH₃) |
| 30 | CF₃CF₂(CF₃)CF | 61 | (CH₃)₂CHOCH₂CH(CH₃) |
| 31 | (CF₃)₂FCCF₂ | 62 | Cyclopropyl C(CH₃)₂ |
| 32 | (CF₃)₃C | 63 | Cyclopropyl CH(CH₃) |
| 33 | (CH₃)₂C=CH | 64 | (CH₃)₂BrC |
| 34 | CH₂=C(CH₃)CH₂ | 65 | (CH₃)₂ClC |
| 35 | C₂H₅CH=CH | 66 | (CH₃)₂FC |
| 36 | HC≡CCH₂ | 67 | (CH₃)₂(CH₃S)C |
| 37 | CH₃C≡CCH₂ | 68 | CH₂ =C(CH₃) |
| 38 | HC≡C(CH₂)₂ 68 | | |
| 39 | CH₃OCH₂ | | |
| 40 | CH₃SCH₂ | | |
| 41 | C₂H₅OCH₂ | | |
| 42 | C₂H₅SCH₂ | | |
| 43 | (CH₃)₂CHOCH₂ | | |
| 44 | (CH₃)₂CHSCH₂ | | |
| 45 | (CH₃)₃COCH₂ | | |
| 46 | (CH₃)₃CSCH₂ | | |
| 47 | (CH₃)₃C(CH₃)₂SiOCH₂ | | |
| 48 | CH₃O(CH₂)₂OCH₂ | | |
| 49 | CH₃O(CH₂)₂ | | |
| 50 | CH₃S(CH₂)₂ | | |
| 51 | C₂H₅O(CH₂)₂ | | |

### TABLE 2

Table 2 comprises 68 compounds of the general formula (I), wherein A is N, B is OCH₃ ana X has the value listed for the correspondingly numbered compound in Table 1.

### TABLE 3

Table 3 comprises 68 compounds of the general formula (I), wherein A is CH, B is NHCH₃ and X has the value listed for the correspondingly numbered compound in Table 1.

### TABLE 4

Table 4 comprises 68 compounds of the general formula (I), wherein A is N, B is NHCH₃ and X has the value listed for the correspondingly numbered compound in Table 1.

**TABLE 5**

| | |
|---|---|
| Table 5 shows melting point or selected proton NMR data obtained at 270 MHz for certain compounds described in Table 1. Chemical shifts are measured at 20°C unless otherwise stated and in ppm from tetramethyl silane. Deuterochloroform was used as solvent unless otherwise stated. The following abbreviations are used: s = singlet d = doublet t = triplet q = quartet m = multiplet dd = double doublet | |

| Compound No | Melting Point (°C)/NMR (ppm) |
|---|---|
| 1 | 93 |
| 2 | 1.29-1.35(3H,t); 2.77-2.85(2H,q); 3.62(3H,s); 3.75(3H,s); 7.32-7.45(4H,m); 7.55(1H,s). |
| 3 | 46-49 |
| 4 | 70-72 |
| 8 | 1.4(9H,s); 3.6(3H,s); 3.72(3H,s); 7.3-7.4(4H,m); 7.56(1H,s). |
| 13 | 0.76(3H,t); 1.32(6H,s); 1.74(2H,q); 3.60(3H,s); 3.72(3H,s); 7.33-7.42(4H,m); 7.56(1H,s). |
| 15 | 1.02(9H,s); 2.70(2H,s); 3.62(3H,s); 3.75(3H,s); 7.33-7.43(4H,m); 7.55(1H,s). |
| 18 | 1.89-1.93 & 2.16-2.20(3H,dd) [87% trans, 13% cis]; 3.61(3H,s); 3.73(3H,s); 6.31-6.39(1H,dd); 6.81-6.95(1H,m); 7.32-7.44(4H,m); 7.54(1H,s). |
| 24 | 3.62(3H,s); 3.76(3H,s); 7.35-7.50(4H,m); 7.58(3H,s). |
| 39 | 3.50(3H,s); 3.62(3H,s); 3.78(3H,s); 4.54(2H,s); 7.37(4H,m); 7.54(1H,s). |
| 55 | 1.62(6H,s); 3.23(3H,s); 3.62(3H,s); 3.75(3H,s); 7.38(4H,m); 7.56(1H,s). |
| 64 | 2.2(6H,s); 3.6(3H,s); 3.75(3H,s); 7.4(4H,m); 7.59(1H,s). |
| 66 | 1.78(3H,d); 3.62(3H,s); 3.74(3H,s); 7.35-7.46(4H,m); 7.55(1H,s). |
| 68 | 2.02(6H,s); 3.62(3H,s); 3.73(3H,s); 7.40(4H,m); 7.58(1H,s). |

Compounds of the present invention can be prepared by reacting together a phenol of the general formula (II), wherein A and B have the meanings given before, with a 1,2,4-thiadiazole derivative of the general formula (III), wherein X has the meaning given before and Y is a suitable leaving group such as methylsulphonyl, tosyl or halo, for example chloro, in a suitable solvent, such as N,N-dimethylformamide, and in the presence of a suitable base, such as potassium carbonate or sodium hydride. Conveniently, the phenol and thiadiazole derivative are dissolved in the solvent at a temperature of about 5°C and the base added. Alternatively, the base is stirred in a solution of the phenol in part of the solvent at a temperature of about 5°C and the thiadiazole added in the remaining solvent. The reaction mixture is then stirred at room temperature for several hours, before extraction of the desired product.

Phenols of the formula (II) can be prepared by methods described in, for example, EP-A-0242081, EP-A-0398692 and GB-A-2249092.

Thiadiazole derivatives of the formula (III) can be prepared by adapting standard literature methods (see, for example, J.Goerdeler, H.Grochopp and U.Sommerlad, Chem. Ber., 1957, 90, 182 and "Advances in Heterocyclic Chemistry" (1965) 5, 119).

1,2,4-Thiadiazoles of the formula (III), where Y is tosyl (p-tolylsulphonyl), may be obtained by the thermolysis of 5-substituted 1,3,4-oxathiazol-2-ones in excess p-tolylsulphonylnitrile conveniently in a hydrocarbon solvent, such as dodecane (see R K Howe and J E Franz, J.Org.Chem., 39, 962 (1974)). The 1,3,4-oxathiazolones can be prepared by reaction of acid amides with chlorocarbonylsulphenyl chloride by known procedures (see, for example, GB-A-1079348).

1,2,4-Thiadiazoles of the formula (III), where Y is methylsulphonyl, may be prepared by the reaction of methyl iodide followed by 3-chloroperoxybenzoic acid with 3-substituted-5-mercapto-1,2,4-thiazoles. The 5-mercapto-1,2,4-thiazoles can be prepared by the reaction of amidines with carbon disulphide and sulphur in the presence of sodium methoxide in methanol (see GB-A-1116198).

Alternatively, the invention compounds of formula (I) may be prepared from a compound of the formula (IV), wherein X has the meaning given before and W is a group which can be converted by standard procedures described in the literature into the group BOC.C=A.OCH₃. For example, where W is the group CH₃CO₂C.C=CH.OH, CH₃CO₂C.C=N.OH or CH₃NHOC.C=N.OH it may be converted to the appropriate group BOC.C=A.OCH₃ by methylation with a compound CH₃Z, wherein Z is a leaving group such as halo or CH₃SO₂.O, in the presence of a convenient base (such as sodium hydride or potassium carbonate). Where W is the phenylglyoxylic acid derivative, CO.CO₂CH₃ or CO.CONHCH₃, it may be converted to the appropriate group BOC.C=A.OCH₃ by treatment with the Wittig reagent Ph₃P=CH.OCH₃, wherein Ph is phenyl, or with methylhydroxylamine.

The compounds (IV) can be prepared by reacting a phenol of the general formula (IIa) with a thiadiazole derivative of the general formula (III), as described above for the phenol (II). The phenol (IIa) can be conveniently prepared from appropriately substituted phenylacetic acid derivatives by methods known in the literature (see, for example, EP-A-0178826, EP-A-0254426, EP-A-0278595, EP-A-0299694 and EP-A-0398692).

The compounds of formula (I), wherein A is CH or N and B is NHCH₃, can also be prepared from the corresponding compounds wherein A is CH or N and B is OH, by methods set out in the literature and in other ways described in EP-A-0398692.

In a further aspect the invention provides processes for preparing compounds of formula (I). The compounds of formula (I) are active fungicides and may be used to control one or more of the following pathogens: Pyricularia oryzae on rice and wheat and other Pyricularia spp. on other hosts; Puccinia recondita, Puccinia striiformis and other rusts on wheat, Puccinia hordei, Puccinia striiformis and other rusts on barley, and rusts on other hosts e.g. turf, rye, coffee, pears, apples, peanuts, sugar beet, vegetables and ornamental plants; Erysiphe graminis (powdery mildew) on barley, wheat, rye and turf and other powdery mildews on various hosts such as Sphaerotheca macularis on hops, Sphaerotheca fuliginea on cucurbits (e.g. cucumber), Podosphaera leucotricha on apple and Uncinula necator on vines; Cochliobolus spp., Helminthosporium spp., Drechslera spp. (Pyrenophora spp.), Rhynchosporium spp., Septoria spp. (including Mycosphaerella graminicola and Leptosphaeria nodorum), Pseudocercosporella hergotrichoides and Gaeumannomyces graminis on cereals (e.g. wheat, barley, rye), turf and other hosts; Cercospora arachidicola and Cercosporidium personatum on peanuts and other Cercospora species on other hosts, for example, sugar beet, bananas, soya beans and rice; Botrytis cinerea (grey mould) on tomatoes, strawberries, vegetables, vines and other hosts and other Botrytis spp. on other hosts; Alternaria spp. on vegetables (e.g. cucumber), oil-seed rape, apples, tomatoes, cereals (e.g. wheat) and other hosts; Venturia spp. (including Venturia inaequalis (scab)) on apples, pears, stone fruit, tree nuts and other hosts; Cladosporium spp. on a range of hosts including cereals (e.g. wheat); Monilinia spp. on stone fruit. tree nuts and other hosts; Didymella spp. on tomatoes, turf, wheat and other hosts; Phoma spp. on oil-seed rape, turf, rice, potatoes, wheat and other hosts; Aspergillus spp. and Aureobasidium spp. on wheat, lumber and other hosts; Ascochyta spp. on peas, wheat, barley and other hosts; Plasmopara viticola on vines; other downy mildews such as Bremia lactucae on lettuce, Peronospora spp. on soybeans, tobacco, onions and other hosts, Pseudoperonospora humuli on hops and Pseudoperonospora cubensis on cucurbits; Pythium spp. on turf and other hosts; Phytophthora infestans on potatoes and tomatoes and other Phytophthora spp. on vegetables, strawberries, avocado, pepper, ornamentals, tobacco, cocoa and other hosts; Thanatephorus cucumeris on rice and turf and other Rhizoctonia species on various hosts such as wheat and barley, vegetables, cotton and turf; Sclerotinia spp. on turf, peanuts, oil-seed rape and other hosts; Sclerotium spp. on turf, peanuts and other hosts; Colletotrichum spp. on a range of hosts including turf, coffee and vegetables; Laetisaria fuciformis on turf; Mycosphaerella spp. on banana, peanut, citrus, pecan, papaya and other hosts; Diaporthe spp. on citrus, soybean, melon, pear, lupin and other hosts; Elsinoe spp. on citrus, vines, olives, pecans, roses and other hosts; Pyrenopeziza spp. on oil-seed rape and other hosts; Oncobasidium theobromae on cocoa causing vascular streak dieback; Fusarium spp., Typhula spp., Microdochium nivale, Ustilago spp., Urocystis spp., Tilletia spp., and Claviceps purpurea on a variety of hosts but particularly wheat, barley, turf and maize; Ramularia spp. on sugar beet and other hosts; post-harvest diseases particularly of fruit (e.g. Pencillium digitatum and P. italicum and Trichoderma viride on oranges, Colletotrichum musae and Gloeosporium musarum on bananas and Botrytis cinerea on grapes); other pathogens on vines, notably Eutypa lata, Guignardia bidwellii, Phellinus igniarus, Phomopsis viticola, Pseudopezicula tracheiphila and Stereum hirsutum; other pathogens on lumber, notably Cephaloascus fragrans, Ceratocystis spp., Ophiostoma piceae, Penicillium spp., Trichoderma pseudokoningii, Trichoderma viride Trichoderma harzianum, Aspergillus niger, Leptographium lindbergi and Aureobasidium pullulans; and fungal vectors of viral diseases e.g. Polymyxa graminis on cereals as the vector of barley yellow mosaic virus (BYMV).

Some of the compositions show a broad range of activities against fungi in vitro. The compounds may move acropetally/locally in plant tissue. Moreover, the compounds may be volatile enough to be active in the vapour phase against fungi on the plant. The invention therefore provides a method of combating fungi which comprises applying to a plant, to a seed of a plant or to the locus of the plant or seed a fungicidally effective amount of a compound as hereinbefore defined, or a composition containing the same.

The compounds may be used directly for agricultural purposes but are more conveniently formulated into compositions using a carrier or diluent. The invention thus provides fungicidal compositions comprising a compound as hereinbefore defined and an acceptable carrier or diluent therefor. It is preferred that all compositions, both solid and liquid formulations, comprise 0.0001 to 95%, more preferably 1 to 85%, for example 1 to 25% or 25 to 60%, of a compound as hereinbefore defined.

When applied the foliage of plants, the compounds of the invention are applied at rates of 0.1g to 10kg, preferably 1g to 8kg, more preferably 10g to 4kg, of active ingredient (invention compound) per hectare.

When used as seed dressings, the compounds of the invention are used at rates of 0.0001g (for example 0.001g or 0.05g) to 10g, preferably 0.005g to 8g, more preferably 0.005g to 4g, of active ingredient (invention compound) per kilogram of seed.

The compounds can be applied in a number of ways. For example, they can be applied, formulated or unformulated, directly to the foliage of a plant, to seeds or to other medium in which plants are growing or are to be planted, or they can be sprayed on, dusted on or applied as a cream or paste formulation, or they can be applied as a vapour or as slow release granules.

Application can be to any part of the plant including the foliage, stems, branches or roots, or to soil surrounding the roots, or to the seed before it is planted, or to the soil generally, to paddy water or to hydroponic culture systems. The invention compounds may also be injected into plants or sprayed onto vegetation using electrodynamic spraying techniques or other low volume methods or applied by land or aerial irrigation systems.

The term "plant" as used herein includes seedlings, bushes and trees. Furthermore, the fungicidal method of the invention includes preventative, protectant, prophylactic, systemic and eradicant treatments.

The fungicidal compounds are preferably used for agricultural and horticultural purposes in the form of a composition. The type of composition used in any instance will depend upon the particular purpose envisaged.

The compositions may be in the form of dustable powders or granules comprising the active ingredient (invention compound) and a solid diluent or carrier, for example, fillers such as kaolin, bentonite, kieselguhr, dolomite, calcium carbonate, talc, powdered magnesia, fuller's earth, gypsum, diatomaceous earth and china clay. Such granules can be preformed granules suitable for application to the soil without further treatment. These granules can be made either by impregnating pellets of filler with the active ingredient or by pelleting a mixture of the active ingredient and powdered filler. Compositions for dressing seed may include an agent (for example, a mineral oil) for assisting the adhesion of the composition to the seed; alternatively the active ingredient can be formulated for seed dressing purposes using an organic solvent (for example, N-methylpyrrol-idone, propylene glycol or N,N-dimethylformamide). The compositions may also be in the form of water dispersible powders or water dispersible granules comprising wetting or dispersing agents to facilitate the dispersion in liquids. The powders and granules may also contain fillers and suspending agents.

The fungicidal compositions may also be in the form of soluble powders or granules, or in the form of solutions in polar solvents.

Soluble powders may be prepared by mixing the active ingredient with a water-soluble salt such as sodium bicarbonate, sodium carbonate, magnesium sulphate or a polysaccharide, and a wetting or dispersing agent to improve water dispersibility/solubility. The mixture may then be ground to a fine powder. Similar compositions may also be granulated to form water-soluble granules. Solutions may be prepared by dissolving the active ingredient in polar solvents such as ketones, alcohols and glycol ethers. These solutions may contain surface active agents to improve water dilution and prevent crystallisation in a spray tank. Emulsifiable concentrates or emulsions may be prepared by dissolving the active ingredient in an organic solvent optionally containing a wetting or emulsifying agent and then adding the mixture to water which may also contain a wetting or emulsifying agent. Suitable organic solvents are aromatic solvents such as alkylbenzenes and alkylnaphthalenes, ketones such as cyclohexanone and methylcyclohexanone, chlorinated hydrocarbons such as chlorobenzene and trichlorethane, and alcohols such as benzyl alcohol, furfuryl alcohol, butanol and glycol ethers.

Aqueous suspension concentrates of largely insoluble solids may be prepared by ball or bead milling with a dispersing agent with a suspending agent included to stop the solid settling.

Fungicidal compositions to be used as sprays may be in the form of aerosols wherein the formulation is held in a container under pressure of a propellant, e.g. fluorotrichloromethane or dichlorodifluoromethane.

The invention compounds can be mixed in the dry state with a pyrotechnic mixture to form a composition suitable for generating in enclosed spaces a smoke containing the compounds. Alternatively, the compounds may be used in micro-encapsulated form. They may also be formulated in biodegradable polymeric formulations to obtain a slow, controlled release of the active substance.

By including suitable additives, for example additives for improving the uptake, distribution, adhesive power and resistance to rain on treated surfaces, the different compositions can be better adapted for various utilities. Other additives may be included to improve the biological efficacy of the various formulations. Such additives can be surface active materials to improve the wetting and retention on surfaces treated with the formulation and also the uptake and mobility of the active material, or additionally can include oil based spray additives, for example, certain mineral oil and natural plant oil (such as soya bean and rape seed oil) additives, or blends of them with other adjuvants.

The invention compounds can be used as mixtures with fertilisers (e.g. nitrogen-, potassium-or phosphorus-containing fertilisers). Compositions comprising only granules of fertiliser incorporating, for example coated with, a compound of formula (I) are preferred. Such granules suitably contain up to 25% by weight of the compound. The invention therefore also provides a fertiliser composition comprising a fertiliser and the compound of general formula (I) or a salt or metal complex thereof.

Water dispersible powders, emulsifiable concentrates and suspension concentrates will normally contain surfactants, e.g. a wetting agent, dispersing agent, emulsifying agent or suspending agent. These agents can be cationic, anionic or non-ionic agents. Suitable cationic agents are quaternary ammonium compounds, for example, cetyltrimethylammonium bromide. Suitable anionic agents are soaps, salts of aliphatic monoesters of sulphuric acid (for example, sodium lauryl sulphate), and salts of sulphonated aromatic compounds (for example, sodium dodecylbenzenesulphonate, sodium, calcium or ammonium lignosulphonate, butylnaphthalene sulphonate, and a mixture of sodium diisopropyl- and triisopropylnaphthalene sulphonates).

Suitable non-ionic agents are the condensation products of ethylene oxide with fatty alcohols such as oleyl or cetyl alcohol, or with alkyl phenols such as octyl- or nonylphenol and octylcresol. Other non-ionic agents are the partial esters derived from long chain fatty acids and hexitol anhydrides, alkyl glucosides, polysaccharides and the lecithins and the condensation products of the said partial esters with ethylene oxide. Suitable suspending agents are hydrophilic colloids (for example, polyvinylpyrrolidone and sodium carboxymethylcellulose), and swelling clays such as bentonite or attapulgite.

Fungicidal compositions for use as aqueous dispersions or emulsions are generally supplied in the form of a concentrate containing a high proportion of the active ingredient, the concentrate being diluted with water before use. These concentrates should preferably be able to withstand storage for prolonged periods and after such storage be capable of dilution with water in order to form aqueous preparations which remain homogeneous for a sufficient time to enable them to be applied by conventional spray equipment. The concentrates may conveniently contain up to 95%, suitably 1-85%, for example 1-25% or 25-60%, by weight of the active ingredient. After dilution to form aqueous preparations, such preparations may contain varying amounts of the active ingredient depending upon the intended purpose, but an aqueous preparation containing 0.0001 to 10%, for example 0.005 to 10%, by weight of active ingredient may be used.

The fungicidal compositions of this invention may contain other compounds having biological activity, e.g. compounds having similar or complementary fungicidal activity or which possess plant growth regulating, herbicidal or insecticidal activity.

By including another fungicide, the resulting composition can have a broader spectrum of activity or a greater level of intrinsic activity than the compound of general formula (I) alone. Further the other fungicide can have a synergistic effect on the fungicidal activity of the compound of general formula (I). Examples of fungicidal compounds which may be included in the composition of the invention are (RS)-1-aminopropylphosphonic acid, (RS)-4-(4-chlorophenyl)-2-phenyl-2-(1H-1,2,4-triazol-1-ylmethyl)butyronitrile, (Z)-N-but-2-enyloxymethyl-2-chloro-2',6'-diethylacetanilide, 1-(2-cyano-2--methoxyiminoacetyl)-3-ethyl urea, 4-(2,2-difluoro-1,3-benzodioxol-4-yl)-pyrrole-3-carbonitrile, 4-bromo-2-cyano-N,N-dimethyl-6-trifluoromethyl-benzimidazole-1-sulphonamide, 5-ethyl-5,8-dihydro-8-oxo(1,3)-dioxol-(4,5-g)quinoline-7-carboxylic acid, α-[N-(3-chloro-2,6-xylyl)-2-methoxyacetamido]-γ -butyrolactone, N-(2-methoxy-5-pyridyl)-cyclopropane carboxamide, alanycarb, aldimorph, ampropylfos, anilazine, azaconazole, BAS 490F, benalaxyl, benomyl, biloxazol, binapacryl, bitertanol, blasticidin S, bromuconazole, bupirimate, butenachlor, buthiobate, captafol, captan, carbendazim, carbendazim chlorhydrate, carboxin, chinomethionate, chlorbenzthiazone, chloroneb, chlorothalonil, chlorozolinate, clozylacon, copper containing compounds such as copper oxychloride, copper oxyquinolate, copper sulphate, copper tallate, and Bordeaux mixture, cycloheximide, cymoxanil, cyproconazole, cyprofuram, debacarb, di-2-pyridyl disulphide 1,1'-dioxide, dichlofluanid, dichlone, diclobutrazol, diclomezine, dicloran, didecyl dimethyl ammonium chloride, diethofencarb, difenoconazole, O,O-di-iso-propyl-S-benzyl thiophosphate, dimefluazole, dimetconazole, dimethomorph, dimethirimol, diniconazole, dinocap, dipyrithione, ditalimfos, dithianon, dodemorph, dodine, doguadine, edifenphos, epoxiconazole, etaconazole, ethirimol, ethoxyquin, ethyl (Z)-N-benzyl-N-([methyl(methyl-thioethylideneamino-oxycarbonyl)amino]thio)-β-alaninate, etridiazole, fenaminosulph, fenapanil, fenarimol, fenbuconazole, fenfuram, fenpiclonil, fenpropidin, fenpropimorph, fentin acetate, fentin hydroxide, ferbam, ferimzone, fluazinam, fludioxonil, fluoroimide, fluquinconazole, flusilazole, flutolanil, flutriafol, folpet, fuberidazole, furalaxyl, furconazole-cis, guazatine, hexaconazole, hydroxyisoxazole, hymexazole, ICIA5504, imazalil, imibenconazole, ipconazole, iprobenfos, iprodione, isopropanyl butyl carbamate, isoprothiolane, kasugamycin, mancozeb, maneb, mepanipyrim, mepronil, metalaxyl, metconazole, methfuroxam, metiram, metiram-zinc, metsulfovax, myclobutanil, neoasozin, nickel dimethyldithiocarbamate, nitrothal-isopropyl, nuarimol, ofurace, organomercury compounds, oxadixyl, oxolinic acid, oxycarboxin, pefurazoate, penconazole, pencycuron, phenazin oxide, phosetyl-Al, phosphorus acids, phthalide, polyoxin D, polyram, probenazole, prochloraz, procymidone, propamocarb, propamocarb hydrochloride, propiconazole, propineb, propionic acid, prothiocarb, pyracarbolid, pyrazophos, pyrifenox, pyrimethanil, pyroquilon, pyroxyfur, pyrrolnitrin, quaternary ammonium compounds, quinconazole, quinomethionate, quintozene, rabenazole, sodium pentachlorophenate, streptomycin, sulphur, SSF-126, tebuconazole, techlofthalam, tecnazene, tetraconazole, thiabendazole, thicyofen, thifluzamide, 2-(thiocyanomethylthio)benzothiazole, thiophanate-methyl, thiram, timibenconazole, tolclofos-methyl, tolylfluanid, triacetate salt of 1,1'-iminodi(octamethylene)diguanidine, triadimefon, triadimenol, triazbutyl, triazoxide, tricyclazole, tridemorph, triforine, triflumizole, triticonazole, validamycin A, vapam, vinclozolin, XRD-563, zineb and ziram. The compounds of general formula (I) can be mixed with soil, peat or other rooting media for the protection of plants against seed-borne, soil-borne or foliar fungal diseases.

The compounds of formula (I) may also be used to combat and control infestations of insect pests and also other invertebrate pests, for example, acarine pests. The insect and acarine pests which may be combated and controlled by the use of the invention compounds include those pests associated with agriculture (which term includes the growing of crops for food and fibre products), horticulture and animal husbandry, forestry, the storage of products of vegetable origin, such as fruit, grain and timber, and also those pests associated with the transmission of diseases of man and animals.

In order to apply the compounds to the locus of the pests they are usually formulated into compositions which include in addition to the insecticidally active ingredient or ingredients of formula I suitable inert diluent or carrier materials, and/or surface active agents. The compositions may also comprise another pesticidal material, for example another insecticide or acaricide, or a fungicide, or may also comprise an insecticide synergist, such as for example dodecyl imidazole, safroxan, or piperonyl butoxide.

The compositions may be in the form of dusting powders wherein the active ingredient is mixed with a solid diluent or carrier, for example kaolin, bentonite, kieselguhr, or talc, or they may be in the form of granules, wherein the active ingredient is absorbed in a porous granular material for example pumice.

Alternatively the compositions may be in the form of baits wherein the active ingredient is mixed with a nutrient carrier for example sucrose, yeast, malt extract, cereal or cereal products and optionally an attractant such as a pheromone or pheromone analogue. Alternatively the compositions may be in the form of liquid preparations to be used as dips or sprays, which are generally aqueous dispersions or emulsions of the active ingredient in the presence of one or more known wetting agents, dispersing agents or emulsifying agents (surface active agents) of the type described above for the fungicidal compositions. The insecticidal compositions may be prepared by dissolving the active ingredient in a suitable solvent, for example, a ketonic solvent such as diacetone alcohol, or an aromatic solvent such as trimethylbenzene and adding the mixture so obtained to water which may contain one or more known wetting, dispersing or emulsifying agents.

Other suitable organic solvents are dimethyl formamide, ethylene dichloride, isopropyl alcohol, propylene glycol and other glycols, diacetone alcohol, toluene, kerosene, white oil, methylnaphthalene, xylenes and trichloroethylene, N-methyl-2-pyrrolidone and tetrahydrofurfuryl alcohol (THFA).

The insecticidal compositions which are to be used in the form of aqueous dispersions or emulsions are generally supplied in the form of a concentrate containing a high proportion of the active ingredient or ingredients, the said concentrate to be diluted with water before use. These concentrates are often required to withstand storage for prolonged periods and after such storage, to be capable of dilution with water to form aqueous preparations which remain homogeneous for a sufficient time to enable them to be applied by conventional spray equipment. The concentrates may contain 10-85% by weight of the active ingredient or ingredients. When diluted to form aqueous preparations such preparations may contain varying amounts of the active ingredient depending upon the purpose for which they are to be used. For agricultural or horticultural purposes, an aqueous preparation containing between 0.0001% and 0.1 % by weight of the active ingredient (approximately equivalent to from 5-2000g/ha) is particularly useful.

In use the insecticidal compositions are applied to the pests, to the locus of the pests, to the habitat of the pests, to growing plants liable to infestation by the pests, or, where there is systemic uptake by plants, to the soil surrounding plants liable to infestation, by any of the known means of applying pesticidal compositions, for example, by dusting or spraying.

The compounds of the invention may be the sole active ingredient of the insecticidal composition or they may be admixed with one or more additional active ingredients such as insecticides, insecticide synergists, herbicides, fungicides or plant growth regulators where appropriate.

Suitable additional active ingredients for inclusion in admixture with the compounds of the invention may be compounds which will broaden the spectrum of activity of the compounds of the invention or increase their persistence in the location of the pest. They may synergise the activity of the compound of the invention or complement the activity for example by increasing the speed of effect, improving knockdown or overcoming repellency.

Additionally multi-component mixtures of this type may help to overcome or prevent the development of resistance to individual components.

The particular insecticide, herbicide or fungicide included in the mixture will depend upon its intended utility and the type of complementary action required. Examples of suitable insecticides include the following:
a) Pyrethroids such as permethrin, esfenvalerate, deltamethrin, cyhalothrin in particular lambda-cyhalothrin, cypermethrin, alpha cypermethrin, bifenthrin, fenpropathrin, cyfluthrin, tefluthrin, fish safe pyrethroids for example ethofenprox, natural pyrethrin, tetramethrin, s-bioallethrin, fenfluthrin, prallethrin, or 5-benzyl-3-furylmethyl-(E)-(1R,3 S)-2,2-dimethyl-3 -(2-oxothiolan-3 -ylidenemethyl) cyclopropane carboxylate;
b) Organophosphates such as profenofos, sulprofos, methyl parathion, azinphos-methyl, demeton-s-methyl, heptenophos, thiometon, fenamiphos, monocrotophos, triazophos, methamidophos, dimethoate, phosphamidon, malathion, chloropyrifos, phosalone, fensulfothion, fonofos, phorate, phoxim, pirimiphos-methyl, fenitrothion or diazinon;
c) Carbamates (including aryl carbamates) such as pirimicarb, cloethocarb, carbofuran, ethiofencarb, aldicarb, thiofurox, carbosulfan, bendiocarb, fenobucarb, propoxur or oxamyl;
d) Benzoyl ureas such as triflumuron, or chlorfluazuron;
e) Organic tin compounds such as cyhexatin, fenbutatin oxide, or azocyclotin;
f) Macrolides such as avermectins or milbemycins, for example such as abamectin, ivermectin, and milbemycin;
g) Hormones such as pheromones;
h) Organochlorine compounds such as benzene hexachloride, DDT, chlordane or dieldrin.
i) Amidines, such as chlordimeform or amitraz.

In addition to the major chemical classes of insecticide listed above, other insecticides having particular targets may be employed in the mixture if appropriate for the intended utility of the mixture. For instance selective insecticides for particular crops, for example stemborer specific insecticides for use in rice such as cartap or buprofezin can be employed. Alternatively insecticides specific for particular insect species/stages for example ovo-larvicides such as clofentezine, flubenzimine, hexythiazox and tetradifon, acaricides such as dicofol, propargite, bromopropylate. chlorobenzilate, or growth regulators such as hydramethylnon, cyromazine, methoprene, hydroprene, chlorfluazuron and diflubenzuron may also be included in the compositions.

Examples of suitable insecticide synergists for use in the compositions include piperonyl butoxide, sesamex, and dodecyl imidazole.

Suitable herbicides, fungicides and plant-growth regulators for inclusion in the compositions will depend upon the intended target and the effect required. An example of a rice selective herbicide which can be included is propanil, an example of a plant growth regulator for use in cotton is "Pix", and examples of fungicides for use in rice include blasticides such as blasticidin-S.

The ratio of the compound of the invention to the other active ingredient in the composition will depend upon a number of factors including type of target, effect required from the mixture etc.

However in general, the additional active ingredient of the composition will be applied at about the rate as it is usually employed, or at a slightly lower rate if synergism occurs. The compounds of formula I and insecticidal compositions comprising them have shown themselves active against a variety of insect and other invertebrate pests. They are particularly useful in controlling sucking pests such as aphids and mites. Compounds of the present invention are also generally characterised by a relatively broad spectrum of activity which may include Lepidoptera and Coleoptera in addition to public health pests such as flies and cockroaches.

They may also be active against organophosphate, pyrethroid or cyclodiene (for example lindane or dieldrin) resistant strains of public and animal health pests. They may be effective in combating both susceptible and resistant strains of the pests in their adult and immature stages of growth, and may be applied to the infested host animal by topical, oral or parenteral administration.

The following Examples illustrate the invention. Throughout the Examples, the term 'ether' refers to diethyl ether, magnesium sulphate was used to dry solutions except where otherwise indicated, and solutions were concentrated under reduced pressure. All reactions were performed under an atmosphere of nitrogen and solvents were dried before use, where appropriate. Unless otherwise stated, chromatography was performed on a column of silica gel as the stationary phase. Where shown, infrared and NMR data are selective; no attempt is made to list every absorption in all cases. ¹H NMR spectra were recorded using CDCl₃ solutions unless otherwise stated. The following abbreviations are used throughtout:

| | |
|---|---|
| NMR = nuclear magnetic resonance | ppm = parts per million |
| s = singlet | IR = infrared |
| m = multiplet | DMF = N,N-dimethylformamide |
| mp = melting point | DMSO = dimethylsulphoxide |

### EXAMPLE 1

This Example illustrates the preparation of (E)-methyl 2-(2-[3-iso-propyl-1,2,4-thiadiazol-5-yloxy]phenyl)-3-methoxycrylate (Compound 4 of Table 1).

A solution of (E)-methyl 2-(2-hydroxyphenyl)-3-methoxyacrylate (0.92g; prepared as described in Example 3 of EP-A-0242081) in DMF (10ml) was added to dried potassuim carbonate (0.61g). A solution of 3-iso-propyl-5-(4methylphenylsulphonyl)-1,2,4-thiadiazole (1.13g) in DMF (10ml) was then added to the stirred mixture during 10 minutes. The reaction mixture was left stirring overnight. The resulting solution was poured into water and extracted with ether (3 times). The combined ether layers were washed twice with dilute sodium hydroxide solution then three times with water before drying. Concentration gave an orange gum. Column chromatography, eluting with 2:1 ether/hexane, gave the title compound as a gum which solidified on standing (0.69g, 49%); mp 70-72°C.

### EXAMPLE 2

### This Example illustrates the preparation of (E)-methyl 2-(2-[3-trifluoromethyl-1,2,4-thiadiazol-5-yloxy]phenyl)-3-methoxyacrylate (Compound 24 of Table 1).

(E)-Methyl 2-(2-hydroxyphenyl)-3-methoxyacrylate (2.12g; prepared as described in Example 3 of EP-A-242081) was added to a stirred solution of 3-trifluoromethyl-5-chloro-1,2,4-thiadiazole (0.96g) in DMF (15ml). The reaction was cooled to 5°C and potassium carbonate (1.4g) was added. After stirring at room temperature for 4 hours the reaction mixture was poured into water and extracted with ether. The ether layers were dried and concentrated to give a yellow oil. Column chromatography, eluting with dichloromethane/hexane 6:1 gave the required product (0.42g, 19%); ¹H NMR 6 3.62(3H,s); 3.76(3H,s); 7.35-7.50(4H,m); 7.58(1H,s) ppm.

### EXAMPLE 3

The compounds were tested against a variety of foliar fungal diseases of plants. The technique employed was as follows.

The plants were grown in John Innes Potting Compost (No 1 or 2) in 4 cm diameter minipots. The test compounds were formulated either by bead milling with aqueous Dispersol T or as a solution in acetone or acetone/ethanol which was diluted to the required concentration immediately falls exactly mid-way between two of the points, it is rounded to the lower of the two values.

The results are shown in Table 6.

**TABLE 6**

| Compound No of Table 1 | Pr | Egt | Sn | Po | Tc | Vi | Pv | Pil |
|---|---|---|---|---|---|---|---|---|
| 1 | 0 | 90 | 30 | 20 | 0 | 0 | - | 0 |
| 2 | 0 | 0 | - | 0 | 0 | 0 | 0 | - |
| 3 | 0 | 0 | 0 | 0 | 0 | 0 | 0 | 90 |
| 4 | 0 | 0 | 0 | 0 | 0 | 0 | 0 | - |
| 8 | 0 | 0 | - | 0 | 0 | 0 | 0 | 0 |
| 13 | 0 | 0 | 0 | - | 0 | 0 | 0 | 0 |
| 15 | - | 90 | 0 | 0 | 60 | 0 | 0 | 0 |
| 18 | 0 | 0 | 0 | 0 | 0 | 0 | 0 | 90 |
| 24 | 0 | 0 | 0 | 0 | 90 | 0 | 0 | 0 |
| 39 | 0 | 0 | 5 | - | - | 0 | 0 | 0 |
| 55 | 0 | 0 | 30 | - | - | 0 | 0 | 0a |
| 64 | 5a | 30a | 0a | 0a | 90a | 0a | 0a | 0a |
| 66 | 0 | 0 | - | 0 | 0 | 0 | 0 | - |
| a = 10ppm foliar application only. Otherwise, data represent activity following application as a combined foliar spray and root drench treatment at 100 ppm. - No result | | | | | | | | |
| Key to Diseases Pr Puccinia recondita Tc Thanetophorus cucumeris Egt Erysiphe graminis tritici Vi Venturia inaequalis Sn Septoria nodorum PvPlasmopara viticola PoPyricularia oryzae Pil Phytophthora infestans lycopersici | | | | | | | | |

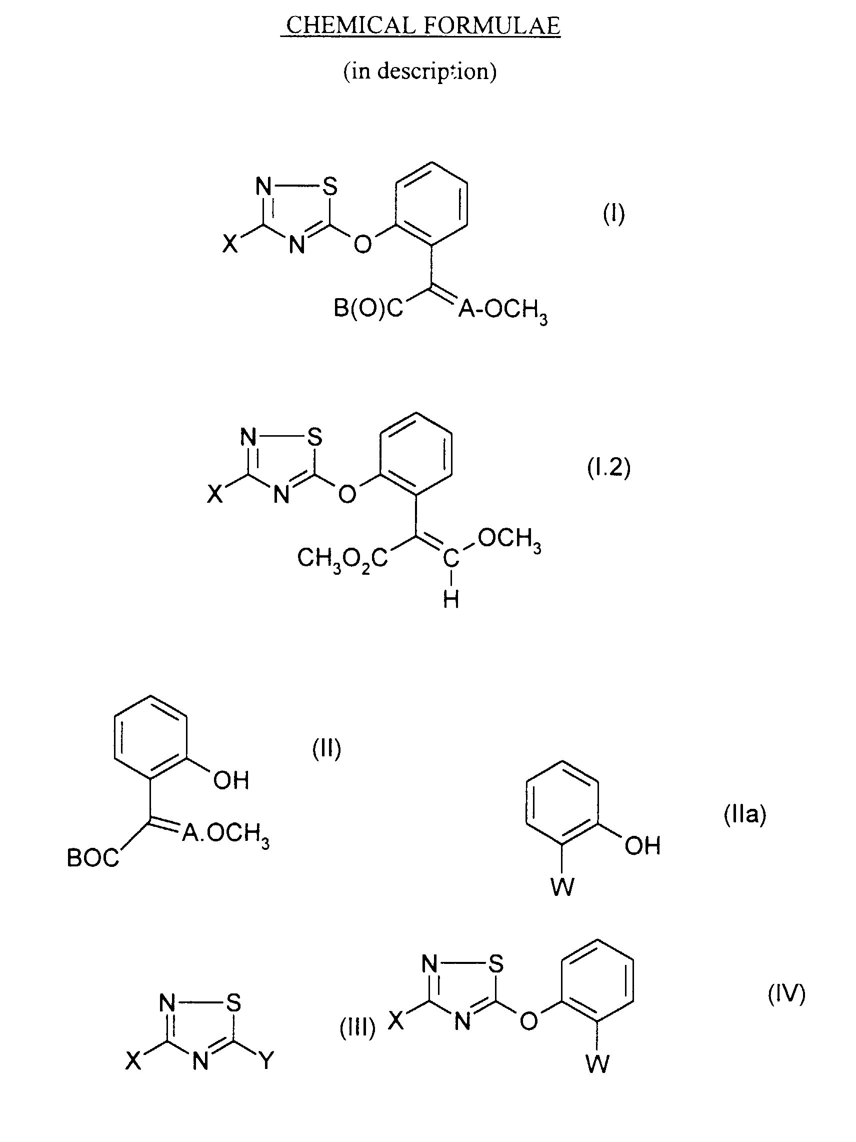

## Claims

1. A compound having the general formula (I): 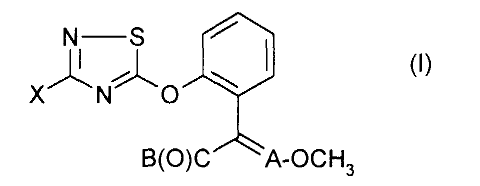 or a stereoisomer thereof, wherein A is CH or N; B is OCH₃ or NHCH₃; and X is C₁₋₈ alkyl (optionally substituted with C₁₋₈ alkoxy, halo(C₁₋₈)alkoxy, C₁₋₈ alkoxy(C₁₋₈)alkoxy, C₁₋₈ alkylthio, C₃₋₆ alkenyloxy, C₃₋₆ alkynyloxy or trialkylsilyloxy), halo(C₁₋₈)alkyl, C₂₋₈ alkenyl, C₂₋₈ alkynyl or C₃₋₇ cycloalkyl(C₂₋₆)alkyl.

2. A compound according to claim 1 wherein X is C₁₋₆ alkyl (optionally substituted with C₁₋₄ alkoxy, C₁₋₂ alkoxy(C₁₋₄)alkoxy or C₁₋₄ alkylthio), halo(C₁₋₆)alkyl, C₃₋₆ alkenyl, C₃₋₄ alkynyl or cyclopropyl(C₂₋₃)alkyl.

3. A compound according to claim 1 or 2 wherein A is N and B is OCH₃ or NHCH₃.

4. A compound according to claim 1 or 2 wherein A is CH and B is OCH₃.

5. The (E)-isomer of a compound according to any one of the preceding claims.

6. A process for preparing compounds according to claim 1 which comprises reacting a phenol of the general formula (IIa): 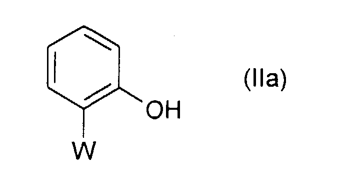 wherein W is the group B(O)C-C=A-OCH₃, in which A and B have the meanings given in claim 1, or a group which is subsequently converted to the group B(O)C-C=A-OCH₃, with a thiadiazole derivative of the general formula (III): 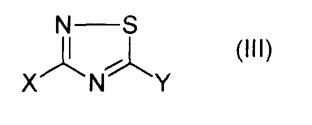 wherein X has the meaning given in claim 1 and Y is a leaving group, in a solvent in the presence of a base.

7. A fungicidal composition comprising a fungicidally effective amount of a compound according to claim 1 and a fungicidally acceptable carrier or diluent therefor.

8. A method of combating fungi which comprises applying to plants, to the seeds of plants or to the locus of the plants or seeds, a compound according to claim 1 or a composition according to claim 7.

## Patentansprüche

1. Verbindung mit der allgemeinen Formel (I) : 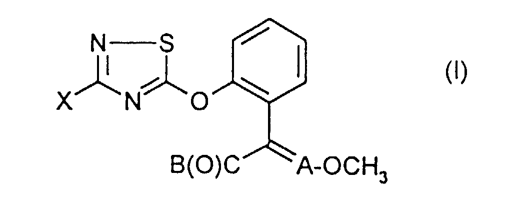 oder ein Stereoisomer davon, worin A CH oder N ist; B OCH₃ oder NHCH₃ ist; und X C₁₋₈-Alkyl (gegebenenfalls substituiert mit C₁₋₈-Alkoxy, Halogen-(C₁₋₈)alkoxy, C₁₋₈-Alkoxy-(C₁₋₈)alkoxy, C₁₋₈-Alkylthio, C₃₋₆-Alkenyloxy, C₃₋₆-Alkinyloxy oder Trialkylsilyloxy), Halogen-(C₁₋₈)alkyl, C₂₋₈-Alkenyl, C₂₋₈-Alkinyl oder C₃₋₇-Cycloalkyl-(C₂₋₆)alkyl ist.

2. Verbindung gemäß Anspruch 1, worin X C₁₋₆-Alkyl (gegebenenfalls substituiert mit C₁₋₄-Alkoxy, C₁₋₂-Alkoxy-(C₁₋₄)alkoxy oder C₁₋₄-Alkylthio), Halogen-(C₁₋₆)alkyl, C₃₋₆-Alkenyl, C₃₋₄-Alkinyl oder Cyclopropyl-(C₂₋₃)alkyl ist.

3. Verbindung gemäß Anspruch 1 oder 2, worin A N ist und B OCH₃ oder NHCH₃ ist.

4. Verbindung gemäß Anspruch 1 oder 2, wortin A CH ist, und B OCH₃ ist.

5. (E)-Isomer einer Verbindung gemäß einem der vorhergehenden Ansprüche.

6. Verfahren zur Herstellung von Verbindungen gemäß Anspruch 1, welches das Umsetzen eines Phenols der allgemeinen Formel (IIa): 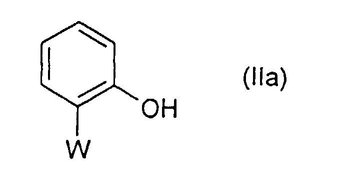 worin W die Gruppe B(O)C-C=A-OCH₃ ist, worin A und B die in Anspruch 1 angegebenen Bedeutungen haben, oder einer Gruppe, die anschließend zur Gruppe B(O)C-C=A-OCH₃ konvertiert wird, mit einem Thiadiazol-Derivat der allgemeinen Formel (III): 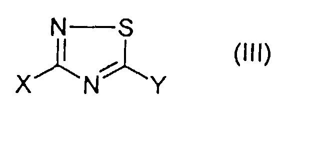 worin X die in Anspruch 1 angegebene Bedeutung hat und Y eine Abgangsgruppe ist, in einem Lösungsmittel in Gegenwart einer Base umfaßt.

7. Fungizide Zusammensetzung, die eine fungizid wirksame Menge einer Verbindung gemäß Anspruch 1 und einen dafür fungizid akzeptablen Träger oder ein Verdünnungsmittel umfaßt.

8. Verfahren zur Bekämpfung von Pilzen, welches die Aufbringung einer Verbindung gemäß Anspruch 1 oder einer Zusammensetzung gemäß Anspruch 7 auf die Samen von Pflanzen oder den Lokus der Pflanzen oder der Samen umfaßt.

## Revendications

1. Composé répondant à la formule générale (I) : 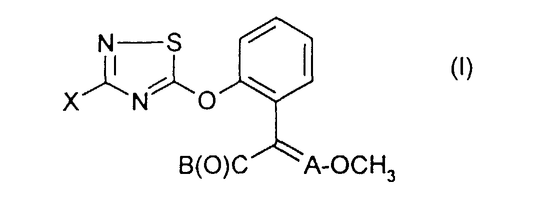 ou un de ses stéréo-isomères, formule dans laquelle A représente un groupe CH ou N ; B représente un groupe OCH₃ ou NHCH₃ ; et X représente un groupe alkyle en C₁ à C₈ (facultativement substitué avec des substituants alkoxy en C₁ à C₈, halogénalkoxy en C₁ à C₈, (alkoxy en C₁ à C₈) (alkoxy en C₁ à C₈), alkylthio en C₁ à C₈, alcényloxy en C₃ à C₆, alcynyloxy en C₃ à C₆ ou trialkylsilyloxy), halogénalkyle en C₁ à C₈, alcényle en C₂ à C₈, alcynyle en C₂ à C₈ ou (cycloalkyle en C₃ à C₇) (alkyle en C₂ à C₆).

2. Composé suivant la revendication 1, dans lequel X représente un groupe alkyle en C₁ à C₆ (facultativement substitué avec des substituants alkoxy en Ci à C₄, (alkoxy en C₁ ou C₂) (alkoxy en C₁ à C₄) ou alkylthio en C₁ à C₄), halogénalkyle en C₁ à C₆, alcényle en C₃ à C₆, alcynyle en C₃ ou C₄ ou cyclopropyl(alkyle en C₂ ou C₃).

3. Composé suivant la revendication 1 ou 2, dans lequel A représente N et B représente un groupe OCH₃ ou NHCH₃.

4. Composé suivant la revendication 1 ou 2, dans lequel A représente un groupe CH et B représente un groupe OCH₃.

5. Isomère(E) d'un composé suivant l'un quelconque des revendications précédentes.

6. Procédé pour la préparation de composés suivant la revendication 1, qui comprend la réaction d'un phénol de formule générale (IIa) : 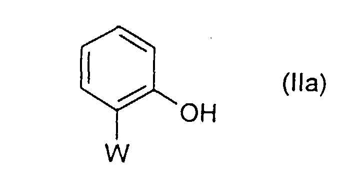 dans laquelle W représente le groupe B(O)C-C=A-OCH₃, dans lequel A et B répondent aux définitions indiquées dans la revendication 1, ou un groupe qui est transformé ultérieurement en le groupe B(O)C-C=A-OCH₃, avec un dérivé de thiadiazole de formule générale (III) : 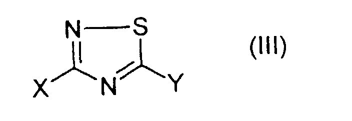 dans laquelle X répond à la définition mentionnée dans la revendication 1 et Y représente un groupe partant, dans un solvant en présence d'une base.

7. Composition fongicide comprenant une quantité, efficace du point de vue fongicide, d'un composé suivant la revendication 1 et un support ou diluant acceptable du point de vue fongicide.

8. Procédé pour combattre des champignons, qui comprend l'application à des plantes, aux semences des plantes ou au milieu dans lequel se trouvent ces plantes ou ces semences d'un composé suivant la revendication 1 ou d'une composition suivant la revendication 7.
